(19) Europäisches Patentamt
European Patent Office
Office européen des brevets

(11) Veröffentlichungsnummer: **0 011 844**
**B1**

(12) # EUROPÄISCHE PATENTSCHRIFT

(45) Veröffentlichungstag der Patentschrift: **26.05.82**

(51) Int. Cl.³: **C 07 C 93/14,** A 61 K 7/13

(21) Anmeldenummer: **79104694.9**

(22) Anmeldetag: **26.11.79**

(54) **Neue Kupplerkomponenten für Oxidationshaarfarben, deren Herstellung sowie diese enthaltende Haarfärbemittel.**

(30) Priorität: **02.12.78 DE 2852156**
**10.08.79 DE 2932460**

(43) Veröffentlichungstag der Anmeldung: **11.06.80 Patentblatt 80/12**

(45) Bekanntmachung des Hinweises auf die Patenterteilung: **26.05.82 Patentblatt 82/21**

(84) Benannte Vertragsstaaten: **BE CH FR GB IT NL SE**

(56) Entgegenhaltungen:
**DE-A-1 719 381**
**DE-A-2 658 329**
**DE-A-2 737 138**

**M.S. Baldham «Cosmetics, Science and Technology», 2. Auflage, Teil 2, 1972, Wiley, N.Y., Seite 298, 306, 307, 308.**

(73) Patentinhaber: **Henkel Kommanditgesellschaft auf Aktien, Postfach 1100 Henkelstrasse 67, D-4000 Düsseldorf 1 (DE)**

(72) Erfinder: **Rose, David Dr., Hobeinweg 7, D-4010 Hilden (DE)**
Erfinder: **Busch, Peter Dr., Gottfried-August-Bürger-Strasse 10, D-4006 Erkrath-Unterbach (DE)**
Erfinder: **Lieske, Edgar, Hunsrückenstrasse 40, D-4000 Düsseldorf (DE)**
Erfinder: **Konrad, Günther, Dr., Feuerbachweg 12, D-4010 Hilden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 011 844 B1

Neu Kupplerkomponenten für Oxidationshaarfarben, deren Herstellung sowie diese enthaltende Haarfärbemittel.

Gegenstand der Erfindung sind neue Haarfärbemittel auf der Basis von Oxidationsfarbstoffen mit einem Gehalt an Bis-(2,4-diaminophenoxy)-alkanen der allgemeinen Formel I

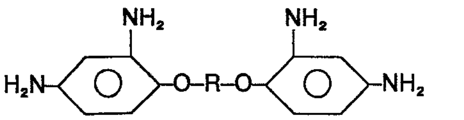 (I)

in der R einen geradkettigen oder verzweigtkettigen Alkylenrest mit 1–12 Kohlenstoffatomen darstellt, oder deren Salzen mit anorganischen oder organischen Säuren als Kupplerkomponenten und den in Oxidationshaarfarben üblichen Entwicklersubstanzen. Ein weiterer Gegenstand der Erfindung sind Bis-(2,4-diaminophenoxy)-alkane der allgemeinen Formel I, in der R einen geradkettigen oder verzweigtkettigen Alkylenrest mit 1–12 Kohlenstoffatomen darstellt, wobei R nicht eine 1,3-Propylengruppe bedeuten soll.

Gegenstand der Erfindung ist weiterhin ein verbessertes Herstellverfahren für Kupplerkomponenten der allgemeinen Formel I.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen wie p-Phenylendiaminderivate, Diaminopyridine, 4-Aminopyrazolonderivate, heterocyclische Hydrazone eingesetzt. Als sogenannte Kupplerkomponenten werden m-Phenylendiaminderivate, Phenole, Naphthole, Resorcinderivate und Pyrazolone genannt.

Gute Oxidationshaarfarbstoffkomponenten müssen in erster Linie folgende Voraussetzungen erfüllen:

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen und sie sollen darüber hinaus in toxikologischer und dermatologischer Hinsicht unbedenklich sein.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

In dem Aufsatz über «The Chemotherapy of Schistosomiasis» von J.N. Ashley, R.F. Collins, M. Davis und N.E. Sirett, J. Chem. Soc, 1958, ist auf Seite 3309 in Tabelle 5 unter Nr. 3 die Verbindung Bis-(2,4-diaminophenoxy)-propan-1,3 beschrieben, ohne dass die Veröffentlichung irgendeinen Bezug zu Oxidationsfärbstoffen oder Haarfärbemitteln aufweist.

Aus der deutschen Offenlegungsschrift 1 719 381 sind Bis-(2-aminophenoxy)-alkane und Bis-(3-aminophenoxy)-alkane als Kuppler für Azofarbstoffe bekannt. Hinweise auf ihre Eignung als Kuppler für Oxidationsfarbstoffe und insbesondere für Haarfärbemittel sind dieser Druckschrift nicht zu entnehmen.

Aus der deutschen Offenlegungsschrift 2 737 138 sind 2,4-Diaminophenolderivate und Färbemittel für Keratinfasern, besonders Haare, bekannt, die solche 2,4-Diaminophenolderivate enthalten. Die aus dieser Druckschrift bekannten Verbindungen enthalten nur einen 2,4-Diaminophenoxy-Rest im Molekül und befriedigen noch nicht im Hinblick auf die Lichtechtheit der damit erzielbaren Anfärbungen. Darüber hinaus besteht ein zunehmendes Interesse an solchen Haarfarbenkomponenten, die gegenüber den bekannten Produkten eine verringerte Giftigkeit aufweisen.

Es wurde nun gefunden, dass man zu Oxidationshaarfarben, die den gestellten Anforderungen in besonders hohem Masse gerecht werden, gelangt, wenn man als Kupplerkomponenten Bis-(2,4-Diaminophenoxy)-Alkane der allgemeinen Formel

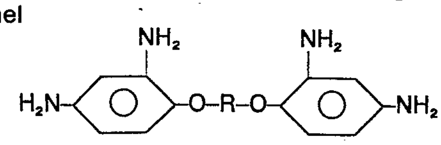

in der R einen geradkettigen oder verzweigtkettigen Alkylenrest mit 1–12 Kohlenstoffatomen darstellt, sowie deren anorganische oder organische Salze in Kombination mit üblichen Entwicklersubstanzen verwendet.

Haarfärbemittel auf Basis von Oxidationsfarbstoffen mit einem Gehalt an Bis-(2,4-Diaminophenoxy)-alkanen der allgemeinen Formel

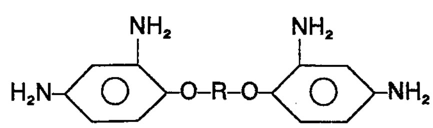

in der R einen geradkettigen oder verzweigtkettigen Alkylenrest mit 1–12 Kohlenstoffatomen bedeutet, sowie deren anorganischen oder organischen Salzen als Kupplerkomponenten und den in Oxidationshaarfarben üblichen Entwicklersubstanzen stellen demnach besonders wertvolle Kompositionen auf dem Gebiet der Oxidationshaarfarben dar.

Besondere Bedeutung ist dabei den Kupplerkomponenten beizumessen, bei denen gemäss vorgenannter Formel, R einen geradkettigen Alkylenrest mit 1–4 Kohlenstoffatomen darstellt.

Bei ihrem Einsatz als Kupplerkomponenten liefern die erfindungsgemässen Verbindungen mit den im allgemeinen für die Oxidationshaarfärbung verwendeten Entwicklersubstanzen sehr intensive, von türkis bis blauschwarz reichende Farbtöne und stellen somit eine wesentliche Bereicherung der oxidativen Haarfärbemöglichkeiten dar. Darüber hinaus zeichnen sich die erfindungsgemässen Bis-(2,4-Diaminophenoxy)-alkane durch sehr gute Echtheitseigenschaften der damit erzielten Färbungen, durch eine gute Löslichkeit in Wasser, eine gute Lagerstabilität und toxikologische sowie dermatologische Unbedenklichkeit aus.

Die in erfindungsgemässen Haarfärbemitteln als Kupplerkomponenten zu verwendenden Bis-(2,4-Diaminophenoxy)-alkane können entweder als solche oder in Form ihrer Salze mit anorganischen oder organischen Säuren, wie z.B. als Chloride, Sulfate, Phosphate, Acetate, Propionate, Lactate, Citrate eingesetzt werden.

Als in erfindungsgemässen Haarfärbemitteln einzusetzende Kupplerkomponenten sind z.B.
Bis-(2,4-Diaminophenoxy)-methan,
1,2-Bis-(2,4-Diaminophenoxy)-ethan,
1,3-Bis-(2,4-Diaminophenoxy)-propan,
1,4-Bis-(2,4-Diaminophenoxy)-butan,
1,5-Bis-(2,4-Diaminophenoxy)-pentan,
1,6-Bis-(2,4-Diaminophenoxy)-hexan,
1,8-Bis-(2,4-Diaminophenoxy)-octan,
1,10-Bis-(2,4-Diaminophenoxy)-decan,
1,12-Bis-(2,4-Diaminophenoxy)-dodecan,
1,2-Bis-(2,4-Diaminophenoxy)-propan,
1,3-Bis-(2,4-Diaminophenoxy)-2-methylpropan,
1,6-Bis-(2,4-Diaminophenoxy)-2-methylhexan
zu nennen.

Zur Herstellung der Bis-(2,4-diaminophenoxy)-alkane bediente man sich bisher üblicherweise eines vierstufigen Verfahrens, welches über nachstehend aufgeführte Stufen verlief:

1. Stufe:
Acetylierung von 2-Amino-4-nitrophenol zu 2-Acetylamino-4-nitrophenol.

2. Stufe:
Umsetzung von 2-Acetylamino-4-nitrophenol mit Dibromalkan zu Bis-(2-acetylamino-4-nitrophenoxy)-alkanen.

3. Stufe:
Abspaltung der Acetylgruppen der Bis-(2-acetylamino-4-nitrophenoxy)-alkane durch Kochen mit Salzsäure zu Bis-(2-amino-4-nitrophenoxy)-alkanen.

4. Stufe:
Katalytische Reduktion der Bis-(2-amino-4-nitrophenoxy)-alkane zu Bis-(2,4-diaminophenoxy)-alkanen.

Die Reaktionen der ersten drei Verfahrensstufen werden näher in der deutschen Offenlegungsschrift 2 658 329 beschrieben, während die Reduktion gemäss der vierten Stufe in Anlehnung an literaturbekannte Hydrierverfahren erfolgt. Ein derartiges vierstufiges Herstellungsverfahren ist jedoch sehr aufwendig, so dass der Wunsch nach einem einfacheren Verfahren bestand.

Es wurde nun ein verbessertes Verfahren zur Herstellung der Bis-(2,4-diaminophenoxy)-alkane der vorgenannten Formel aufgefunden, bei dem man in erster Stufe 2,4-Dinitrophenolat-anion, hergestellt durch Zugabe einer Base zu 2,4-Dinitrophenol, in einem geeigneten Lösungsmittel mit einem Dibromalkan der Formel Br–R–Br, in der R die vorgenannte Bedeutung besitzt, bei Temperaturen zwischen 80–200 °C innerhalb von ½–12 Stunden umsetzt, das so gebildete Bis-(2,4-dinitrophenoxy)-alkan durch Waschen mit einer Base von 2,4-Dinitrophenol befreit und dieses Bis-(2,4-dinitrophenoxy)-alkan in zweiter Stufe in bekannter Weise durch katalytische Hydrierung in das entsprechende Bis-(2,4-diaminophenoxy)-alkan überführt.

Als Basen zur Überführung des 2,4-Dinitrophenols in das 2,4-Dinitrophenolat-anion haben sich Kaliumcarbonat und Kaliumhydroxid am besten bewährt. Geeignete Lösungsmittel für die Durchführung der Reaktion stellen Dimethylformamid, Methylglykol, Diethylenglykolmonomethylether und Diethylenglykoldimethylether dar. Die Umsetzung wird zweckmässigerweise bei Temperaturen zwischen 100–150 °C durchgeführt. Als Katalysator bei der Hydrierung in zweiter Stufe dienen vornehmlich Raney-Nickel oder Palladium auf Kohle.

Mit dem erfindungsgemässen Verfahren wird eine einfache technische Herstellung der vorstehend näher beschriebenen Bis-(2,4-diaminophenoxy)-alkane ermöglicht.

Als Beispiel für in den erfindungsgemässen Haarfärbemitteln einzusetzende Entwicklerkomponenten sind primäre aromatische Amine mit einer weiteren in p-Stellung befindlichen funktionellen Gruppe wie p-Phenylendiamin, p-Toluylendiamin, p-Aminophenol, N-Methyl-p-phenylendiamin, N,N-Dimethyl-p-phenylendiamin, N,N-Diethyl-2-methyl-p-phenylendiamin, N-Ethyl-N-hydroxyethyl-p-phenylendiamin, Chlor-p-phenylendiamin, N,N-Bis-hydroxyethylamino-p-phenylendiamin, Methoxy-p-phenylendiamin, 2,6-Dichlorp-phenylendiamin, 2-Chlor-6-brom-p-phenylendiamin, 2-Chlor-6-methyl-p-phenylendiamin, 6-Methoxy-3-methyl-p-phenylendiamin, andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1–4 Kohlenstoffatomen darstellt, ferner Diaminopyridinderivate, heterocyclische Hydrazonderivate wie 1-Methyl-pyrrolidon-(2)-hydrazon, 4-Aminopyrazolonderivate wie 4-Amino-1-phenyl-3-carbamoylpyrazolon-5, N-Butyl-N-sulfobutyl-p-phenylendiamin, Tetraaminopyrimidine wie 2,4,5,6-Tetraaminopyrimidin, 4,5-Diamino-2,6-bismethylaminopyrimidin, 2,5-Diamino-4-diethylamino-6-pyrimidin, 2,4,5-Triamino-6-dimethylaminopyrimidin, 2,4,5-Triamino-6-piperidinopyrimidin, 2,4,5-Triamino-6-anilino-pyrimidin, 2,4,5-Triamino-6-morpholino-pyrimidin, 2,4,5-Triamino-6-β-hydroxy-ethylamino-pyrimidin anzuführen.

Zur Erzielung möglichst kräftiger und den natürlichen Haarfarbennuancen weitgehend entsprechender Farbtöne ist ein vollwertiger Blaufarbstoff als Nuancierkomponente von besonderer Wichtigkeit. Bei der Erstellung natürlicher Farbnuancen

unter Zuhilfenahme von Blaukupplerkomponenten ergeben sich mit den üblichen Blaukupplern häufig Schwierigkeiten. Diesem Mangel kann durch den Einsatz der erfindungsgemäss zu verwendenden Bis-(2,4-Diaminophenoxy)-alkane weitgehend abgeholfen werden. Diese Kupplerkomponenten liefern mit entsprechenden Entwicklersubstanzen neben anderen Farbnuancen dunkelblaue bis schwarzblaue, besonders intensive Haarfärbungen, die sich durch ausserordentliche Lichtechtheiten auszeichnen.

In den erfindungsgemässen Haarfärbemitteln werden die Kupplerkomponenten im allgemeinen in etwa molaren Mengen, bezogen auf die verwendeten Entwicklersubstanzen, eingesetzt. Wenn sich auch der molare Einsatz als zweckmässig erweist, so ist es jedoch nicht nachteilig, wenn die Kupplerkomponente in einem gewissen Überschuss oder Unterschuss zum Einsatz gelangt.

Es ist ferner nicht erforderlich, dass die Entwicklerkomponente und die Kupplersubstanz einheitliche Produkte darstellen, vielmehr können sowohl die Entwicklerkomponente Gemische der erfindungsgemäss zu verwendenden Entwicklerverbindungen als auch die Kupplersubstanz Gemische der erfindungsgemäss einzusetzenden Bis-(2,4-Diaminophenoxy)-alkane darstellen.

Darüber hinaus können die erfindungsgemässen Haarfärbemittel gegebenenfalls übliche direktziehende Farbstoffe im Gemisch enthalten, falls dies zur Erzielung gewisser Farbnuancen erforderlich ist.

Die oxidative Kupplung, d.h. die Entwicklung der Färbung, kann grundsätzlich wie bei anderen Oxidationshaarfarbstoffen auch, durch Luftsauerstoff erfolgen. Zweckmässigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Die in erfindungsgemässen Haarfärbemitteln zu verwendenden Bis-(2,4-Diaminophenoxy)-alkane besitzen ferner den Vorteil, dass sie bereits bei oxidativer Kupplung durch Luftsauerstoff befriedigende Färbeergebnisse liefern und somit eine Haarschädigung durch das sonst für die oxidative Kupplung eingesetzte Oxidationsmittel vermieden werden kann.

Wird jedoch gleichzeitig neben der Färbung ein Aufhelleffekt am Haar gewünscht, so ist die Mitverwendung von Oxidationsmitteln erforderlich.

Die erfindungsgemässen Haarfärbemittel werden für den Einsatz in entsprechende kosmetische Zubereitungen wie Cremes, Emulsionen, Gelen oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzliche Bestandteile sind zum Beispiel Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Ethylenoxid an Fettalkohole, Verdickungsmittel wie Methylcellulose, Stärke, höhere Fettalkohole, Paraffinöl, Fettsäuren, ferner Parfümöle und Haarpflegemittel wie Pantothensäure und Cholesterin zu nennen. Die genannten Zusatzstoffe werden dabei in den für diese Zwecke üblichen Mengen eingesetzt, wie zum Beispiel Netz- und Emulgiermittel in Konzentrationen von 0,5–30 Gewichtsprozent und Verdickungsmittel in Konzentrationen von 0,1–25 Gewichtsprozent, jeweils bezogen auf die gesamte Zubereitung.

Die Anwendung der erfindungsgemässen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8–10 erfolgen. Die Anwendungstemperaturen bewegen sich dabei im Bereich von 15–40 °C. Nach einer Einwirkungsdauer von ca. 30 Minuten wird das Haarfärbemittel vom zu färbenden Haar durch Spülen entfernt. Hernach wird das Haar mit einem milden Shampoo nachgewaschen und getrocknet.

Die mit den erfindungsgemässen Haarfärbemitteln erzielbaren Blautöne zeigen unter Einsatz unterschiedlicher Entwickler- und Kupplerkomponenten besonders intensive Farbnuancen. Die erzielten Färbungen haben gute Licht-, Wasch- und Reibechtheitseigenschaften und lassen sich leicht mit Reduktionsmitteln wieder abziehen.

Die nachfolgenden Beispiele sollen den Erfindungsgegenstand näher erläutern, ohne ihn jedoch hierauf zu beschränken.

## Beispiele

Zunächst wird nachstehend die Herstellung der erfindungsgemässen Bis-(2,4-Diaminophenoxy)-alkane sowohl nach dem bisher üblichen als auch nach dem verbesserten Herstellungsverfahren beschrieben.

Die Herstellung der als Zwischenprodukte dienenden Bis-(4-Nitro-2-aminophenoxy)-alkane erfolgte gemäss den Angaben in der deutschen Offenlegungsschrift 2 658 329 durch Umsetzung von 2 Mol des entsprechend substituierten Nitroacetaminophenols mit etwas mehr als 1 Mol Dibromalkan in Gegenwart von etwas weniger als 2 Mol Alkalihydroxid in wässrig-alkoholischem Medium bei Temperaturen zwischen 100 und 150 °C zu den Bis-Nitroacetaminoverbindungen und anschliessende Abspaltung der Acetylreste.

### A) Bis-(2,4-Diaminophenoxy)-methan-tetrahydrochlorid

10,4 g Bis-(4-Nitro-2-aminophenoxy)-methan wurden in 200 ml Ethanol und 20 ml Dimethylformamid in Gegenwart von 1,0 g Raney-Nickel bei 80 °C und 100 atü hydriert. Nach beendeter Wasserstoffaufnahme wurde vom Katalysator abfiltriert und zur Trockne eingeengt. Der Rückstand wurde in verdünnter Salzsäure gelöst und die erhaltene Lösung wurde wiederum zur Trockne eingeengt. Der Rückstand wurde bei 70 °C getrocknet. Es wurden 7,8 g Bis-(2,4-Diaminophenoxy)-methan-tetrahydrochlorid erhalten. Die Verbindung schmilzt bei 210 °C unter Zersetzung.

B) 1,2-Bis-(2,4-Diaminophenoxy)-ethan-tetrahydrochlorid

Entsprechend den Angaben unter A wurden 11,5 g 1,2-Bis-(4-Nitro-2-amino-phenoxy)-ethan in 300 ml Ethanol und 150 ml Wasser in Gegenwart von 1,0 g Hydrierkatalysator (5% Palladium auf Kohle) hydriert und aufgearbeitet. Die Ausbeute an 1,2-Bis-(2,4-Diaminophenoxy)-ethan-tetrahydrochlorid vom Schmelzpunkt 179–181 °C betrug 12, g.

C) 1,3-Bis-(2,4-Diaminophenoxy)-propan-tetrahydrochlorid

Entsprechend den Angaben unter A wurden 10,6 g 1,3-Bis-(4-Nitro-2-aminophenoxy)-propan in 150 ml Dimethylformamid in Gegenwart von 1,0 g Raney-Nickel hydriert und aufgearbeitet. Die Ausbeute an 1,3-Bis-(2,4-Diaminophenoxy)-propan-tetrahydrochlorid betrug 9,1 g. Die Verbindung schmolz bei 165 °C unter Zersetzung.

D) 1,4-Bis-(2,4-Diaminophenoxy)-butan-tetrahydrochlorid

Entsprechend den Angaben unter A wurden 8,8 g 1,4-Bis-(4-Nitro-2-aminophenoxy)-butan in 150 ml Dimethylformamid in Gegenwart von 1,0 g Raney-Nickel hydriert und aufgearbeitet. Die Ausbeute an 1,4-Bis-(2,4-Diaminophenoxy)-butan-tetrahydrochlorid betrug 8,2 g. Die Verbindung schmolz bei 222–240 °C unter Zersetzung.

1. Herstellung von 1,3-Bis-(2,4-diaminophenoxy)-propan-tetrahydrochlorid nach dem verbesserten Verfahren

Stufe 1:

Herstellung von 1,3-Bis-(2,4-dinitrophenoxy)-propan.

9,2 g 2,4-Dinitrophenol, angefeuchtet mit 0,6 g Wasser, wurden in 15 ml Diethylenglykoldimethylether suspendiert. Zu der Suspension wurden bei ca. 25 °C 3,45 g Kaliumcarbonat gegeben. Die Suspension wurde hernach auf 100 °C erhitzt und es wurden 5 g 1,3-Dibrompropan zugetropft. Anschliessend wurde 4 Stunden lang auf 125 °C erhitzt. Nach dem Abkühlen wurde der erhaltene Niederschlag abgesaugt, mit Natronlauge und anschliessend mit Wasser gewaschen. Nach dem Trocknen hatte das erhaltene 1,3-Bis-(2,4-dinitrophenoxy)-propan einen Schmelzpunkt von 186 °C. Die Ausbeute betrug 8,2 g, das sind 80% der Theorie.

Stufe 2:

Herstellung von 1,3-Bis-(2,4-diaminophenoxy)-propantetrahydrochlorid.

10 g 1,3-Bis-(2,4-dinitrophenoxy)-propan wurden in 50 ml Ethanol in Gegenwart von Raney-Nickel bei 25 atü und 25 °C hydriert. Nach beendeter Wasserstoffaufnahme wurde vom Katalysator abfiltriert. Die Lösung wurde mit konz. Salzsäure angesäuert und zur Trockne eingeengt. Das 1,3-Bis-(2,4-diaminophenoxy)-propantetrahydrochlorid wurde in Gestalt weisser Kristalle vom Schmelzpunkt 215 °C mit 95%iger Ausbeute der Theorie erhalten.

2. Herstellung von 1,3-Bis-(2,4-diaminophenoxy)-propantetrahydrochlorid

Stufe 1:

Herstellung von 1,3-Bis-(2,4-dinitrophenoxy)-propan.

Die Herstellung erfolgte völlig analog den Angaben in Beispiel 1, nur dass anstelle von Kaliumcarbonat zu der Suspension von 2,4-Dinitrophenol in Diethylenglykoldimethylether 2,8 g Kaliumhydroxid gegeben wurden. Die Ausbeute an 1,3-Bis-(2,4-dinitrophenoxy)-propan betrug 6,6 g, das sind 64% der Theorie.

Stufe 2:

Entsprach völlig der Stufe 2 von Beispiel 1.

3. Herstellung von 1,3-Bis-(2,4-diaminophenoxy)-propan-tetrahydrochlorid

Stufe 1:

Die Herstellung von 1,3-Bis-(2,4-dinitrophenoxy)-propan erfolgte analog den Angaben in Beispiel 1, wobei jedoch als Lösungsmittel 15 ml Methylglykol eingesetzt wurden. Die Ausbeute an 1,3-Bis-(2,4-dinitrophenoxy)-propan betrug 6,6 g, das sind 64% der Theorie.

Stufe 2:

Die Herstellung des 1,3-Bis-(2,4-diaminophenoxy)-propan-tetrahydrochlorids erfolgte analog der Stufe 2 von Beispiel 1.

4. Herstellung von 1,3-Bis-(2,4-diaminophenoxy)-propan-tetrahydrochlorid

Stufe 1:

Bei der analog der Stufe 1 von Beispiel 1 vorgenommenen Herstellung von 1,3-Bis-(2,4-dinitrophenoxy)-propan wurde bei diesem Beispiel als Lösungsmittel Diethylenglykolmonomethylether eingesetzt. Die Ausbeute an gewünschtem Zwischenprodukt betrug 7,5 g, das sind 74% der Theorie.

Stufe 2:

Die weitere Verarbeitung zum Endprodukt erfolgte völlig analog zur Stufe 2 von Beispiel 1.

5. Herstellung von 1,3-Bis-(2,4-diaminophenoxy)-propan-tetrahydrochlorid

Stufe 1:

Die Herstellung von 1,3-Bis-(2,4-dinitrophenoxy)-propan wurde entsprechend der Stufe 1 von Beispiel 1 mit 15 ml Dimethylformamid als Lösungsmittel durchgeführt. Es wurden 5,1 g an gewünschtem Produkt erhalten, das sind 50% der theoretischen Ausbeute.

Stufe 2:

Die weitere Verarbeitung zum 1,3-Bis-(2,4-diaminophenoxy)-propan-tetrahydrochlorid erfolgte analog Stufe 2 von Beispiel 1.

Die vorgenannten, in ihrer Herstellung beschriebenen Bis-(2,4-Diaminophenoxy)-alkane wurden als Kupplerkomponenten in den folgenden Beispielen eingesetzt.

Als Entwicklerkomponenten dienten folgende Substanzen:

E1: 2,4,5,6-Tetraaminopyrimidin
E2: p-Toluylendiamin
E3: p-Phenylendiamin
E4: 2,5-Diaminoanisol
E5: N,N-Bis-(β-hydroxyethyl)-p-phenylendiamin
E6: 2-Anilino-4-hydroxy-5,6-diaminopyrimidin
E7: N,N-Diethyl-2-methyl-p-phenylendiamin
E8: 2-Chlor-p-phenylendiamin
E9: N-Butyl-N-sulfobutyl-p-phenylendiamin
E10: 1-Phenyl-3-carbamoyl-4-amino-pyrazolon.

Die erfindungsgemässen Haarfärbemittel wurden in Form einer Cremeemulsion eingesetzt. Dabei wurden in eine Emulsion aus

10 Gew.-Teilen Fettalkoholen der Kettenlänge $C_{12}$–$C_{18}$,
10 Gew.-Teilen Fettalkoholsulfat (Natriumsalz) Kettenlänge $C_{12}$–$C_{18}$ und
75 Gew.-Teilen Wasser

jeweils 0,01 Mol der in der nachstehenden Tabelle aufgeführten Entwicklersubstanzen und Kupplersubstanzen eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde entweder mit Luftsauerstoff oder mit 1%iger Wasserstoffperoxidlösung als Oxidationsmittel durchgeführt, wobei zu 100 Gewichtsteilen der Emulsion 10 Gewichtsteile Wasserstoffperoxidlösung gegeben wurden. Die jeweilige Färbecreme mit oder ohne Zusatz von Oxidationsmitteln wurde auf zu 90% ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschliessend getrocknet. Die dabei erhaltenen Färbungen sind nachstehender Tabelle 1 zu entnehmen.

Tabelle 1

| Beispiel | Entwickler | Kuppler | Erhaltener Farbton bei Luftoxidation | mit 1%iger $H_2O_2$-Lösung |
|---|---|---|---|---|
| 1 | E1 | A | türkis | dunkeltürkis |
| 2 | E2 | A | blauschwarz | blauschwarz |
| 3 | E3 | A | blauschwarz | blauschwarz |
| 4 | E1 | B | grautürkis | mattblau |
| 5 | E2 | B | mattblau | schwarzblau |
| 6 | E4 | B | dunkelblau | dunkelblau |
| 7 | E5 | B | grautürkis | dunkelblau |
| 8 | E6 | B | mattviolett | dunkelviolett |
| 9 | E7 | B | dunkeltürkis | dunkelblau |
| 10 | E8 | B | violettblau | dunkelviolett |
| 11 | E9 | B | mattgrün | grautürkis |
| 12 | E10 | B | grauviolett | dunkelviolett |

**Patentansprüche**

1. Haarfärbemittel auf Basis von Oxidationsfarbstoffen mit einem Gehalt an Bis-(2,4-diaminophenoxy)-alkanen der allgemeinen Formel I

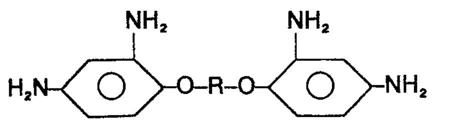 (I)

in der R einen geradkettigen oder verzweigtkettigen Alkylrest mit 1–12 Kohlenstoffatomen darstellt oder deren Salzen mit anorganischen oder organischen Säuren als Kupplerkomponenten und den in Oxidationshaarfarben üblichen Entwicklersubstanzen.

2. Haarfärbemittel nach Anspruch 1, dadurch gekennzeichnet, dass der Gehalt an Entwickler-Kuppler-Kombination 0,2 bis 5 Gewichtsprozent, bezogen auf das gesamte Haarfärbemittel, beträgt.

3. Bis-(2,4-diaminophenoxy)-alkane der allgemeinen Formel I

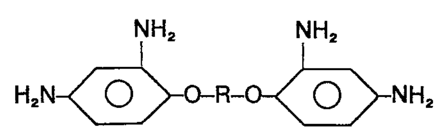 (I)

in der R einen geradkettigen oder verzweigtkettigen Alkylenrest mit 1–12 Kohlenstoffatomen darstellt, wobei R nicht eine 1,3-Propylengruppe bedeuten soll.

4. Verfahren zur Herstellung der Bis-(2,4-diaminophenoxy)-alkane der Formel I gemäss Anspruch 3, dadurch gekennzeichnet, dass man in erster Stufe 2,4-Dinitrophenolat-anion, hergestellt durch Zugabe einer Base zu 2,4-Dinitrophenol, in einem geeigneten Lösungsmittel mit einem

Dibromalkan der Formel Br–R–Br, in der R die im Anspruch 3 genannte Bedeutung besitzt, bei Temperaturen zwischen 80–200 °C innerhalb von ½–12 Stunden umsetzt, das so gebildete Bis-(2,4-dinitrophenoxy)-alkan durch Waschen mit einer Base vom 2,4-Dinitrophenol befreit und dieses Bis-(2,4-dinitrophenoxy)-alkan in zweiter Stufe in bekannter Weise durch katalytische Hydrierung in das entsprechende Bis-(2,4-diaminophenoxy)-alkan überführt.

5. Verfahren nach Anspruch 4, dadurch gekennzeichnet, dass als Base zur Herstellung des Phenolat-anions Kaliumcarbonat oder Kaliumhydroxid eingesetzt wird.

6. Verfahren nach Anspruch 4, dadurch gekennzeichnet, dass als geeignetes Lösungsmittel Dimethylformamid, Methylglykol, Diethylenglykolmonomethylether oder Diethylenglykoldimethylether eingesetzt wird.

7. Verfahren nach Anspruch 4, dadurch gekennzeichnet, dass die Umsetzung bei Temperaturen zwischen 100–150 °C durchgeführt wird.

8. Verfahren nach Anspruch 4, dadurch gekennzeichnet, dass als Katalysator bei der Hydrierung in zweiter Stufe Raney-Nickel oder Palladium auf Kohle verwendet wird.

**Claims**

1. A hair dye based on oxidation dyes and containing bis-(2,4-diaminophenoxy)-alkanes corresponding to the following general formula

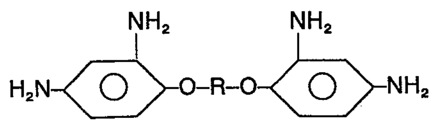

(I)

in which R represents a straight-chain or branched-chain alkyl radical containing from 1 to 12 carbon atoms or their salts with inorganic or organic acids as coupler components and the developer substances normally used in oxidation hair dyes.

2. A hair dye as claimed in Claim 1, characterised in that the content of developer-coupler combination amounts to between 0,2 and 5% by weight, based on the hair dye as a whole.

3. Bis-(2,4-diaminophenoxy)-alkanes corresponding to the following general formula

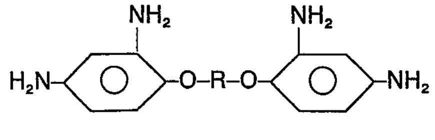

(I)

in which R represents a straight-chain or branched-chain alkylene radical containing from 1 to 12 carbon atoms and is not intended to represent a 1,3-propylene group.

4. A process for producing the bis-(2,4-diaminophenoxy)-alkanes of formula I claimed in Claim 3, characterised in that, in a first stage, 2,4-dinitrophenolate anion, produced by adding a base to 2,4-dinitrophenol, is reacted with a dibromoalkane of the formula Br–R–Br, in which R is as defined in Claim 3, in a suitable solvent for 30 minutes to 12 hours at temperatures in the range from 80 to 200 °C, the bis-(2,4-dinitrophenoxy)-alkane thus formed is freed from the 2,4-dinitrophenol by washing with a base and, in a second stage, this bis-(2,4-dinitrophenoxy)-alkane is converted into the corresponding bis-(2,4-diaminophenoxy)-alkane in known manner by catalytic hydrogenation.

5. A process as claimed in Claim 4, characterised in that potassium carbonate or potassium hydroxide is used as the base for producing the phenolate anion.

6. A process as claimed in Claim 4, characterised in that dimethyl formamide, methyl glycol, diethylene glycol monomethyl ether or diethylene glycol dimethyl ether is used as the solvent.

7. A process as claimed in Claim 4, characterised in that the reaction is carried out at temperatures in the range from 100 to 150 °C.

8. A process as claimed in Claim 4, characterised in that Raney nickel or palladium on carbon is used as the hydrogenation catalyst in the second stage.

**Revendications**

1. Agents de teinture pour cheveux à base de colorants d'oxydation ayant une teneur en bis-(2,4-diaminophénoxy)-alcanes de formule générale I:

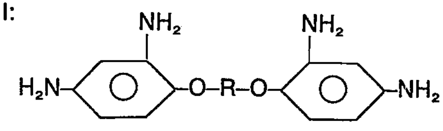

(I)

dans laquelle R représente un radical alcoylène à chaîne droite ou ramifiée ayant 1 à 12 atomes de carbone ou leurs sels avec des acides minéraux ou organiques, en tant que composants de couplage, et en substances de développement qui sont en usage dans les teintures de cheveux par oxydation.

2. Agents de teinture selon la revendication 1, caractérisés en ce que la teneur en la combinaison agent de développement-coupleur s'élève à 0,2–5% en poids par rapport à l'agent de teinture pour cheveux global.

3. Bis-(2,4-diaminophénoxy)-alcanes de formule générale I:

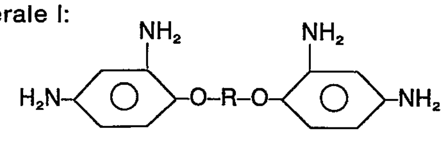

(I)

dans laquelle R représente un radical alcoylène à chaîne droite ou ramifiée ayant 1 à 12 atomes de carbone, R ne devant pas signifier un groupe 1,3-propylène.

4. Procédé de fabrication des bis-(2,4-diaminophénoxy)-alcanes de formule I selon la revendication 3, caractérisé en ce que dans un premier stade on fait réagir un anion 2,4-dinitrophénolate, préparé par addition d'une base à du 2,4-dinitrophénol, dans un solvant approprié, avec un dibromoalcane de formule Br–R–Br, dans laquelle R possède la signification indiquée à la

revendication 3, à des températures entre 80 et 200 °C en l'espace de ½ à 12 heures, on débarrasse le bis-(2,4-dinitrophénoxy)-alcane ainsi formé par lavage avec une base du 2,4-dinitrophénol et, dans une seconde phase, on convertit ce bis-(2,4-dinitrophénoxy)-alcane de manière connue en bis-(2,4-diaminophénoxy)-alcane correspondant par hydrogénation catalytique.

5. Procédé selon la revendication 4, caractérisé en ce qu'en tant que base pour la préparation de l'anion phénolate on utilise du carbonate de potassium ou de l'hydroxyde de potassium.

6. Procédé selon la revendication 4, caractérisé en ce qu'on utilise en tant que solvant approprié de la diméthylformamide, du méthylglycol, de l'éther monométhylique de diéthylène glycol ou de l'éther diméthylique de diéthylène glycol.

7. Procédé selon la revendication 4, caractérisé en ce qu'on exécute la réaction à des températures entre 100 et 150 °C.

8. Procédé selon la revendication 4, caractérisé en ce qu'en tant que catalyseur dans l'hydrogénation au cours du second stade on utilise du nickel de Raney ou du palladium sur charbon.